# EUROPÄISCHE PATENTSCHRIFT

(11) **EP 0 940 397 B1**
(45) Veröffentlichungstag und Bekanntmachung des Hinweises auf die Patenterteilung: **21.11.2001**
(21) Anmeldenummer: 99101872.2
(22) Anmeldetag: 28.01.1999
(51) Int. Cl.: C07D 315/00, C07D 321/00, C07D 313/00

(54) **Verfahren zur Herstellung von makrocyclischen Estern oder Lactonen**
Process for the preparation of macrocyclic esters or lactons
Procédé de préparation d'esters macrocycliques ou de lactones

(30) Priorität: 03.03.1998 DE 19808845
(43) Veröffentlichungstag der Anmeldung: 08.09.1999
(73) Patentinhaber: Degussa AG, 40474 Düsseldorf (DE)
(72) Erfinder: Kohler, Günther Dr., 45770 Marl (DE); Chlench, Jürgen, 46286 Dorsten (DE)

(56) Entgegenhaltungen:
- CH-A- 205 521
- GB-A- 2 004 552
- US-A- 4 709 058

## Beschreibung

Die Erfindung betrifft ein verbessertes zweistufiges Verfahren zur Herstellung von makrocyclischen Estern oder Lactonen aus Dicarbonsäuren oder Dicarbonsäuredialkylestern und Diolen bzw. aus Hydroxycarbonsäuren unter Verwendung eines neuen Katalysators. Die Erfindung betrifft weiterhin die zweite Stufe des Verfahrens für sich allein.

Makrocyclische Ester und Lactone spielen aufgrund ihrer bekannten Moschus- oder Ambranote und darüber hinaus als Fixateure in der Riechstoffindustrie eine bedeutende Rolle. Der größte Bedarf besteht an 13- bis 18-gliedrigen Ringsystemen.

Es ist bekannt, daß makrocyclische Lactone durch eine zweistufige Reaktion hergestellt werden können, in der man zunächst einen linearen Oligo- oder Polyester herstellt, der dann thermisch depolymerisierend cyclisiert wird. Die depolymerisierende Cyclisierung ist z.B. in J.Am.Chem.Soc. 57 (1935). 929-34 und in US-A-4,175,321 beschrieben.

Die Verfahren zur Herstellung der Oligo- oder Polyester in der ersten Stufe, die von Dicarbonsäuren und Diolen ausgehen, arbeiten in der Regel bei 120 bis 200°C und zumeist ohne Katalysator. Beispielsweise begnügt man sich nach DE 32 25 431 mit der Acidität der Dicarbonsäure unter Verzicht auf einen sauren Katalysator und nimmt dabei lange Reaktionszeiten in Kauf. Die Verfahren nach der DOS 28 40 905 und der US-Patentschrift 4,393,223 verwenden einen sauren Festbettkatalysator. Vor der Depolymerisationsstufe muß jedoch ein weiterer Katalysator hinzugegeben werden, weil der saure Festbettkatalysator in der nachfolgenden Depolymerisationsstufe wenig oder unvollkommen wirksam ist.

Die cyclisierende Depolymerisation der zweiten Stufe ist z.B. in J.Am.Chem.Soc. 57 (1935), 929-34 und in US-A-4,175,321 beschrieben. Sie wird in Gegenwart eines Katalysators und im allgemeinen bei Temperaturen von 200 bis 300°C durchgeführt. Die sich bildenden Makrocyclen besitzen unter diesen Bedingungen einen relativ hohen Dampfdruck, so daß sie durch Destillation aus dem Reaktionsgemisch bzw. dem Reaktor entfernt werden können. Eine große Zahl von Katalysatoren für diese Stufe ist bekannt, z.B. Lewis-Säuren, Alkali- bzw. Erdalkalialkoholate, Oxide, Hydroxide und Salze, beispielsweise der Halogenwasserstoffsäuren. Salpetersäure, Kohlensäure. Borsäure und von Carbonsäuren. Auch ein Teil der Säuren, die den Salzen zugrunde liegen, ist als Katalysator geeignet. Als kationischer Bestandteil der Salze kommen Alkalimetalle, Erdalkalimetalle und Schwermetalle in Betracht, wie Eisen, Kupfer, Blei, Zink, Zinn, Nickel, Titan und Zirconium.

Bei Verfahren, die von Dicarbonsäuredialkylestern und Diolen ausgehen, also den Oligo- oder Polyester durch Umesterung herstellen, haben sich als Katalysatoren Alkylstannate, -titanate und -zirconate bewährt (siehe DE 32 254 31, DE 28 409 05). Sie eignen sich auch als Katalysatoren für die depolymerisierende Cyclisierung. Diese Verbindungen haben jedoch einen relativ hohen Dampfdruck und sind von den makrocyclischen Estern oder Lactonen nur schwierig abzutrennen. Da sie zugleich Umesterungskatalysatoren sind, führt ihre Anwesenheit selbst in Konzentrationen von wenigen ppm bei der Destillation oder der Lagerung zu linearen Oligomeren oder Polymeren.

Es wurde nun gefunden, daß sich makrocyclische Ester oder Lactone in einer zweistufigen Reaktion durch lineare Oligomerisierung von Monomeren mit entsprechender Zahl von Kohlenstoffatomen und cyclisierende Deoligomerisierung vorteilhaft herstellen lassen, wenn man beide Stufen in Gegenwart eines Chelat-Komplexes des dreiwertigen Eisens durchführt.

Das Verfahren liefert in hervorragenden Ausbeuten makrocyclische Ester oder Lactone, die durch Destillation im Vakuum in sehr reiner, zur Verwendung in der Riechstoffindustrie geeigneter Form erhalten werden. Der Katalysator ist unter den Reaktionsbedingungen nicht flüchtig, so daß die Reindestillation nicht durch katalytische Rückreaktion zu offenkettigen Oligo- oder Polyestern gestört wird. Die cyclisierende Deoligomerisierung der Oligoester in der zweiten Stufe läßt sich elegant mit deren Herstellung in der ersten Stufe verbinden, da die Chelat-Komplexe des dreiwertigen Eisens sowohl Veresterungs- als auch Umesterungskatalysatoren sind.

Die makrocyclischen Ester oder Lactone können 10 bis 24 Ringglieder umfassen. Bevorzugt werden die erwähnten, in der Riechstoffindustrie geschätzen Makrocyclen mit 13 bis 18 Ringgliedern. Die bevorzugten makrocyclischen Lactone mit 13 bis 18 Ringgliedern erhält man aus omega-Hydroxycarbonsäuren mit 12 bis 17 Kohlenstoffatomen. Alternativ erhält man makrocyclische Ester (oder Dilactone) aus Dicarbonsäuren oder Dicarbonsäuredialkylestern und Diolen, wobei diese Monomeren so ausgewählt werden müssen, daß Dilactone mit der gewünschten Ringgliederzahl entstehen können. Dilactone mit 14 Ringgliedern erhält man z.B. aus linearen Oligoestern, die aus Adipinsäure und 1.6-Hexandiol aufgebaut sind. Dodecandisäure und Ethylenglykol ergeben ein makrocyclisches Dilacton mit 16 Ringgliedern. Die makrocyclischen Ester oder Lactone können aus Monomeren aufgebaut sein, die. von den Hydroxyl- und Carboxylgruppen abgesehen, gesättigte Kohlenwasserstoffstruktur aufweisen. Sie können aber auch Sauerstoff oder Stickstoffatome enthalten.

Für das erfindungsgemäße Verfahren geeignete Hydroxycarbonsäuren haben z.B. 8 bis 20 Kohlenstoffatome zwischen den funktionellen Gruppen. Beispiele hierfür sind u.a. omega-Hydroxycarbonsäuren, wie 11-Hydroxydodecansäure, 12-Hydroxytridecansäure, 15-Hydroxyhexadecansäure, 17-Hydroxyoctadecansäure und 11-Hydroxy-10-methyldodecansäure. Geeignete Dicarbonsäuren haben beispielsweise 4 bis 20 Kohlenstoffatome zwischen den Carboxylgruppen. Beispiele hierfür sind u.a. Bernsteinsäure, Adipinsäure, Korksäure, Sebacinsäure und 1,12-Dodecandisäure. Von den geeigneten Diolen, die z.B. 2 bis 12 Kohlenstoffatome zwischen den Hydroxylgruppen haben können, seien z.B. Ethylenglykol, Ethylendiglykol, 1,3-Propandiol, 1,4-Butandiol und 1,6-Hexandiol. 1,8-Octandiol und 1,12-Dodecandiol genannt. Wie erwähnt, können die Makrocyclen neben den aus der Kondensationsreaktion von Carboxyl- und Hydroxylgruppen stammenden Sauerstoffatomen weitere Heteroatome im Ring enthalten. Dafür werden dann DicarbonSäuren, Diole und/oder Lactone mit den entsprechenden Heteroatomen eingesetzt.

Ein wesentliches Merkmal des erfindungsgemäßen Verfahrens ist die Verwendung eines Eisen (III)-chelatkomplexes als Katalysator. Besonders wirksam sind die Eisen(III)-chelat-Komplexe, bei denen die Chelatkomplexbildner zweizähnige Liganden sind. Von den geeigneten Chelatkomplexbildnern seien beispielsweise 1,3-Dicarbonylverbindungen, wie Acetylaceton und Acetessigsäureester: Ethylendiamintetraessigsäure (EDTA), Nitrilotriessigsäure (NTA). Kaliumhexacyanoferrat(III) und Ethylenglykol-bis-(2-aminoethyl)-tetraessigsäure genannt. Bevorzugt werden NTA sowie deren saure oder neutrale Alkalisalze. Die Eisen(III)-Komplexe sind bekannte Stoffe und werden, zweckmäßig in wäßrigem Medium, direkt aus Eisen(III)-salzen und den Komplexbildnern hergestellt. Man setzt sie vorteilhaft in Mengen von 0,1 bis 1 Gewichtsprozent ein, bezogen auf die Ausgangsstoffe für die linearen Oligomeren, und zwar als einmalige Charge zu Beginn der Umsetzung in der ersten Stufe, in zwei Anteilen jeweils zu Beginn der Umsetzung in den beiden Stufen oder aber in kleineren Anteilen oder kontinuierlich über die beiden Stufen verteilt.

In der ersten Stufe des erfindungsgemäßen Verfahrens werden die Monomeren in lineare Oligoester umgewandelt, die vorzugsweise ein (durchschnittliches) Molekulargewicht von etwa 400 bis etwa 10.000 haben, bestimmt durch Gelpermeationschromatographie mit Polystyrol als Standard. Man arbeitet je nach den Ausgangsstoffen und der Katalysatormenge zweckmäßig bei einer Temperatur von 50 bis 300°C. unter Atmosphärendruck, vermindertem oder erhöhtem Druck von 0,01 hPa bis 10 MPa. Zur Verbesserung der Reaktionsführung oder der Löslichkeit der Reaktionspartner kann ein inertes Lösemittel mitverwendet wer den. Bei der Herstellung von makrocyclischen Estern (oder Dilactonen) kann man in bekannter Weise durch Variation des Molverhältnisses von Dicarbonsäure zu Diol das Molekulargewicht regeln. Zweckmäßig wendet man das Diol in größerem molaren Überschuß, bespielsweise von 3:1 bis 20:1 an. Stellt man makrocyclische Lactone aus Hydroxycarbonsäuren her, so kann man das Molekulargewicht durch die Reaktionsdauer und/oder durch Verdünnung mit einem hochsiedenden inerten Lösemittel, z.B. einem Polyethylenglykoldialkylether, regeln. Für ein gegebenes System lassen sich die Parameter, mit denen ein gewünschtes Molekulargewicht erreicht werden kann, durch orientierende Versuche unschwer bestimmen.

In der ersten Stufe des Verfahrens kann man auch statt des Eisen(III)-Chelat-Komplexes einen üblichen sauren Katalysator, z.B. einen sauren anorganischen oder organischen Ionenaustauscher einsetzen, den man vor dem Eintritt in die zweite Stufe entfernt, z.B. durch Abfiltrieren. Man muß dann zwar einen Katalysatorwechsel vornehmen, hat aber gegenüber den erwähnten, bisher bevorzugten Zinn-, Titan- und Zirconiumkatalysatoren immer noch den Vorteil, daß man keine nachträgliche Polymerisation des makrocyclischen Reaktionsproduktes befürchten muß.

In der zweiten Stufe des erfindungsgemäßen Verfahrens erhitzt man den linearen Oligoester in Gegenwart des Eisen (III)-Chelat-Komplexes auf eine Temperatur von 150 bis 300°C, zweckmäßig unter einem Druck von 0,01 bis 700 hPa. Druck und Temperatur werden so korreliert, daß der entstehende Makrocyclus und etwaige Lösemittel sowie etwaige überschüssige Reaktionspartner, z.B. Diol, abdestillieren. Aus dem Destillat kann man durch erneute Destillation die makrocyclische Verbindung in hoher Reinheit gewinnen.

Man kann die Umsetzung in beiden Stufen oder in einer der beiden Stufen absatzweise oder kontinuierlich durchführen. Bei absatzweiser Herstellung arbeitet man z.B. in einem Rührkessel mit aufgesetzter Destillationskolonne. Kontinuierlich kann man die Reaktion durchführen, indem man die Reaktanten und den Katalysator unter zweckentsprechendem Druck durch entsprechend temperierte Reaktionszonen führt und das Reaktionsgemisch kontinuierlich aufdestilliert.

Die folgenden Beispiele sollen die Erfindung weiter erläutern, nicht jedoch deren Anwendungsbereich begrenzen.

### Beispiel 1

### 1.1 Herstellung des Oligoesters

In einem 2 1-Glaskolben mit Rührer und Destillationsaufsatz werden 300 g Dodecandisäuredimethylester (1,16 mol) und 600 g Ethylenglykol (9,7 mol) sowie 0,3 g Mono-Na-Salz des Eisen(III)-Ethylendiamintetraessigsäure-Komplexes vorgelegt. Das Gemisch wird auf 180°C erhitzt. Dabei destillieren unter Normaldruck innerhalb von 3 bis 4 h ca. 70 bis 74 g Methanol ab. Die Umsetzung kann mittels Gaschromatographie verfolgt werden. Auf diese Weise wird ein Oligoester erhalten, der einen Schmelzbereich von 40 bis 50°C und ein durchschnittliches Molekulargewicht von ca. 600 aufweist.

### 1.2 Cyclisierungsstufe

Das Reaktionsgemisch der ersten Stufe wird nun langsam bis auf 250°C erhitzt. Gleichzeitig wird der Druck nach und nach auf 15 hPa gesenkt. Dabei destillieren Ethylenglykol und das cyclische Ethylenglykoldodekandioat ab. Durch weitere Destillation des Destillats erhält man 180 g reines Ethylenglykoldodecandioat, entsprechend einer Ausbeute von 95%.

### Beispiel 2

### 2.1 Herstellung des Oligoesters

In einem 2 l-Glaskolben mit Rührer und Destillationsaufsatz werden 586 g Brassylsäuredimethylester (2,15 mol) und 294 g Ethylenglykol (4,74 mol) sowie 1,6 g Tris-(2,5-pentadianato)eisen(III) (0,005 mol) erhitzt. Die Reaktionstemperatur wird 4 h im Bereich von 150°C bis 250°C gehalten. Dabei destillieren unter Normaldruck innerhalb von 3 bis 4 h ca. 134 g Methanol (ca. 4,2 mol) ab. Man erhält 746 g Oligoester mit einem mittleren Molekulargewicht von ca. 600 und einem Schmelzbereich von 40 bis 50°C.

### 2.2 Cyclisierungsstufe

In einem 1 l-Glaskolben mit Rührer und Destillationsaufsatz werden 300 g Polyethylenglykoldimethylether mit einem Molekulargewicht von ca. 2.000 vorgelegt und bei einem Druck von 10 hPa auf 280°C erhitzt. Dazu dosiert man innerhalb von 8 h 746 g des auf 170°C temperierten Oligoesters, der mit 1.500 g Ethylenglykol vermischt ist. Kurz nach Beginn der Zudosierung destillieren Ethylenbrassylat und Ethylenglykol als Gemisch ab. Dieses Gemisch trennt sich in zwei Phasen. Die obere, Ethylenbrassylat enthaltende Phase wird einer Reindestillation unterzogen. Man erhält 540 g reines Ethylenbrassylat. Die untere, Ethylenglykol enthaltende Phase aus der Phasentrennung wird in die erste Stufe zur Herstellung des Oligoesters zurückgeführt. Die Ausbeute beträgt 92 %d.Th., bezogen auf eingesetzten Brassylsäuredimethylester.

### Beispiel 3

### 3.1 Herstellung des Oligoesters

In einem 2 1 Glaskolben werden 399 g Dodecandisäure (12.1 mol), 1.653 g Ethylenglykol (26,6 mol) sowie 1,65 g Kalium-hexacyanoferrat(III) (0,05 mol) vorgelegt und 4 h bei 170 bis 200°C gerührt. Man leitet einen schwachen Stickstoffstrom durch das Gemisch, aus dem ca. 33 g Wasser auskondensieren. Der auf diese Weise hergestellte Oligoester hat ein mittleres Molekulargewicht von ca. 800 und einen Schmelzpunkt von 50 bis 55°C.

### 3.2 Cyclisierungsstufe

In einem 1 l-Glaskolben mit Rührer und Destillationsaufsatz werden 300 g Polyethylenglykoldimethylether mit einem Molekulargewicht von ca. 2.000 vorgelegt und bei einem Druck von 1 hPa auf 260°C erhitzt. Dazu dosiert man innerhalb von 4 h 1.017 g des Oligoesters. Kurz nach Beginn der Zudosierung destilliert das cyclische Ethylenglykoldodecanoat zusammen mit Ethylenglykol über. Dieses Gemisch trennt sich in zwei Phasen. Die obere, Ethylenglykoldodecanoat enthaltende Phase wird einer Reindestillation unterzogen. Man erhält 314 g reines Ethylenglykoldodecandioat, entsprechend einer Ausbeute von 95% d.Th., bezogen auf eingesetzte Dodecandisäure.

### Beispiel 4

In einer ersten Stufe wird ein Oligoester der 15-Hydroxypentadecansäure mit einem durchschnittlichen Oligomerisierungsgrad von 3.5 hergestellt, wobei das Mono-Na-Salz des Eisen(III)-Komplexes der Ethylendiamintetraessigäure als Katalysator verwendet wird. In der zweiten Stufe werden in einem 1 Reaktor 240 g Oligoester, die 0.5 g des genannten Na-Salzes enthalten, zusammen mit 300 g Polyethylenglykoldiethylether mit einem mittleren Molekulargewicht von ca. 2.000 vorgelegt. Das Gemisch wird bei einem Druck von 2 hPa auf 300°C erhitzt. Nach ca. 4 h sind 214 g Cyclopentadecanolid abdestilliert, was einer Ausbeute von ca. 89 % d.Th., bezogen auf eingesetzte 15-Hydroxypentadecansäure, entspricht.

## Patentansprüche

1. Verfahren zur Herstellung von makrocyclischen Estern oder Lactonen in einer zweistufigen Reaktion durch lineare Oligomerisierung von Monomeren mit einer dem Makrocyclus entsprechenden Zahl von Kohlenstoffatomen und cyclisierende Deoligomerisierung,
**dadurch gekennzeichnet,**
**dass** man die cyclisierende Deoligomerisierung in Gegenwart von Chelatkomplexen des dreiwertigen Eisens durchführt.

2. Verfahren nach Anspruch 1,
**dadurch gekennzeichnet,**
**dass** man die lineare Oligomerisierung und die cyclisierende Deoligomerisierung in Gegenwart von Chelatkomplexen des dreiwertigen Eisens durchführt.

3. Verfahren nach Anspruch 1 oder 2,
**dadurch gekennzeichnet,**
**dass** als Chelatkomplex ein Komplex des dreiwertigen Eisens mit Acetylaceton, Acetessigsäureester, Ethylendiamintetraessigsäure (EDTA), Nitrilotriessigsäure (NTA) oder Ethylenglykol-bis-(2-aminoethyl)-tetraessigsäure oder Kaliumhexacyanoferrat(III) eingesetzt wird.

4. Verfahren nach Anspruch 1 oder 2
**dadurch gekennzeichnet,**
**dass** die makrocyclische Verbindung 13 bis 18 Ringglieder hat.

5. Verfahren nach einem der Ansprüche 1 bis 4,
**dadurch gekennzeichnet,**
**dass** der lineare Oligoester bei 50 bis 300 °C und einem Druck von 0,01 hPa bis 10 MPa hergestellt wird.

6. Verfahren nach einem der Ansprüche 1 bis 5,
**dadurch gekennzeichnet,**
**dass** die deoligomerisierende Cyclisierung bei einer Temperatur von 150 bis 300 °C unter einem Druck von 0,01 bis 700 hPa durchgeführt wird.

7. Verfahren nach einem der Ansprüche 1 bis 6,
**dadurch gekennzeichnet,**
**dass** die Konzentration des Chelatkomplexes des dreiwertigen Eisens 0,01 bis 1 Gew.-%, bezogen auf die Ausgangsstoffe der ersten Stufe, beträgt.

8. Verfahren nach einem der Ansprüche 1 bis 6,
**dadurch gekennzeichnet,**
**dass** es absatzweise oder kontinuierlich durchgeführt wird.

## Claims

1. A process for the preparation of macrocyclic esters or lactones in a two-stage reaction by linear oligomerization of monomers having a number of carbon atoms corresponding to the macrocycle and cyclizing deoligomerization, **characterized in that** the cyclizing deoligomerization is carried out in the presence of chelate complexes of trivalent iron.

2. A process according to claim 1, **characterized in that** the linear oligomerization and the cyclizing deoligomerization are carried out in the presence of chelate complexes of trivalent iron.

3. A process according to claim 1 or 2, **characterized in that** a complex of trivalent iron with acetylacetone, acetoacetic ester, ethylenediaminetetraacetic acid (EDTA), nitrilotriacetic acid (NTA) or ethylene glycol bis(2-aminoethyl)tetra-acetic acid or potassium hexacyanoferrate(III) is used as chelate complex.

4. A process according to claim 1 or 2, **characterized in that** the macrocyclic compound has 13 to 18 ring members.

5. A process according to any one of claims 1 to 4, **characterized in that** the linear oligoester is prepared at 50 to 300°C and a pressure of from 0.01 hPa to 10 MPa.

6. A process according to any one of claims 1 to 5, **characterized in that** the deoligomerizing cyclization is carried out at a temperature of from 150 to 300°C at a pressure of from 0.01 to 700 hPa.

7. A process according to any one of claims 1 to 6, **characterized in that** the concentration of the chelate complex of trivalent iron is from 0.01 to 1% by weight, based on the starting materials in the first stage.

8. A process according to any one of claims 1 to 6, **characterized in that** it is carried out batchwise or continuously.

## Revendications

1. Procédé de préparation d'esters ou de lactones macrocycliques dans une réaction en deux stades, par oligomérisation linéaire de monomères avec un nombre correspondant au macrocycle, d'atomes de carbone et désoligomérisation cyclisante,
**caractérisé en ce qu'**
on effectue la désoligomérisation cyclisante en présence de complexes chélatés du fer trivalent.

2. Procédé selon la revendication 1,
**caractérisé en ce qu'**
on effectue l'oligomérisation linéaire et la désoligomérisation cyclisante en présence de complexes chélatés du fer trivalent.

3. Procédé selon la revendication 1 ou la revendication 2,
**caractérisé en ce qu'**
on met en oeuvre comme complexe chélaté, un complexe du fer trivalent avec l'acétylacétone, l'acétate d'éthyle, l'acide éthylène diamine tétraacétique (EDTA), l'acide nitrilotriacétique (NTA) ou l'acide éthylèneglycol bis (2-aminoéthyl)-tétraacétique ou l'hexacyanoferrate (III) de potassium.

4. Procédé selon la revendication 1 ou la revendication 2,
**caractérisé en ce que**
le composé macrocyclique a de 13 à 18 éléments de cycle.

5. Procédé selon l'une quelconque des revendications 1 à 4,
**caractérisé en ce que**
l'oligoester linéaire est préparé à 50 à 300°C et à une pression allant de 0,01 hpa à 10 MPa.

6. Procédé selon l'une quelconque des revendications 1 à 5,
**caractérisé en ce que**
la cyclisation désoligomérisante est effectuée à une température allant de 150 à 300°C, sous une pression allant de 0,01 à 700 hPa.

7. Procédé selon l'une quelconque des revendications 1 à 6,
**caractérisé en ce que**
la concentration du complexe chelaté du fer trivalent est de 0,01 à 1 % en poids rapporté aux matières premières de la première étape.

8. Procédé selon l'une quelconque des revendications 1 à 6,
**caractérisé en ce qu'**
on l'effectue graduellement ou en continu.
